# EUROPEAN PATENT SPECIFICATION

(11) **EP 0 419 569 B1**
(45) Date of publication and mention of the grant of the patent: **06.09.1995**
(21) Application number: 89907594.9
(22) Date of filing: 15.06.1989
(51) Int. Cl.: A61K 38/00, A61K 39/04

(54) **STRESS PROTEINS AND USES THEREFOR**
STRESSPROTEINE UND VERWENDUNGEN DAFÜR
PROTEINES DE STRESS ET LEURS UTILISATIONS

(30) Priority: 15.06.1988 US 207298
(43) Date of publication of application: 03.04.1991
(73) Proprietor: WHITEHEAD INSTITUTE FOR BIOMEDICAL RESEARCH, Cambridge, MA 02142 (US); MEDICAL RESEARCH COUNCIL, London W1N 4AL (GB)
(72) Inventor: YOUNG, Richard, A., Winchester, MA 01890 (US); YOUNG, Douglas, Middlesex HA4 6ED (GB)
(74) Representative: Price, Vincent Andrew
(86) International application number: US8902619
(87) International publication number: WO8912455

(56) References cited:
- EP-A- 0 262 170
- WO-A-85/05034
- WO-A-88/00974
- WO-A-88/05823
- WO-A-88/06591
- US-B- 4 724 144
- Welsh et al J. Biol. Chem. 1983 258:7102-7111
- Proc. Natl. Acad. Sci, USA, vol. 85, June 1988, D. Young et al., pages 4267-4270.
- Proc. Natl. Acad. Sci, USA, vol. 84, March 87. R. N. Husson et al. pages 1679-1683.
- Nature, vol. 316, 01.08.85, R.A. Young et al., pages 450-452
- Infection and Immunity, vol. 55, n. 6, 1987, American Society for Microbiology, J.E.R. Thole et al., pages 1466-1475.
- Vodkin and Williams (1988), J. Bact. 170(3) pp.1227-1234
- Ardeshir et al. (1987), EMBO Journal Vol. 6 N. 2 pp. 493-499

## Description

### Background of the Invention

Although the function of stress proteins is not entirely clear, it appears that some participate in assembly and structural stabilization of certain cellular and viral proteins, and their presence at high concentrations may have an additional stabilizing effect during exposure to adverse conditions. Neidhardt, F.C. and R.A. VanBogelen, In: Escherichia coli and Salmonella typhimurium, Cellular and Molecular Biology, (eds. Neidhardt, F.C., Ingraham, J.L., Low, K.B., Magasanik, B. Schaechter, M. and Umbarger, H.E. (Am. Soc. Microbiol., Washington, D.C.), pp. 1334-1345 (1987); Pelham, H.R.B. Cell, 46:959-961 (1986); Takano, T. and T. Kakefuda, Nature, 239:34-37 (1972); Georgopoulos, C. et al., New Biology, 239:38-41 (1972). Phagocytic host cells produce a hostile environment for foreign organisms, and the ability to produce stress proteins has been implicated in the survival of bacterial pathogens within macrophages Christman, M.F. et al., Cell, 41:753-762 (1985).

Mycobacterium (M.) tuberculosis and Mycobacterium (M.) leprae are the etiologic agents of tuberculosis and leprosy, respectively. These diseases afflict 20-30 million people and continue to present a significant global health problem. Joint International Union Against Tuberculosis and World Health Organization Study Group, Tubercle, 63:157-169 (1982); Bloom, B. and T. Godal, Rev. Infect Dis. 5:765-780 (1983). To develop more effective tools for the diagnosis and prevention of these diseases, it is important to understand the immune response to infection by mycobacterial pathogens.

The antibody and T-cell responses to infection or inoculation with killed mycobacteria have been studied in humans and in animals. Human patients with tuberculosis or leprosy produce serum antibodies directed against at least 12 mycobacterial proteins. Some of these proteins are also recognized by well-characterized murine monoclonal antibodies. Mice immunized with mycobacterial lysates produce antibodies that are directed predominantly to six M. tuberculosis and six M. leprae protein antigens. Engers, H.D., Infect. Immun., 48:603-605 (1985); Engers, H.D., Infect. Immun., 51:718-720 (1986). Genes encoding these 12 mycobacterial antigens have been cloned, and recombinant proteins produced from these clones have been used to investigate the human T-lymphocyte response to mycobacterial infection. Husson, R.N. and R.A. Young, Proc. Natl. Acad. Sci., USA, 84:1679-1683 (1987); Young, R.A. et al., Nature, 316: 450-452 (1985); Britton, W.J. et al., Lepr. Rev., 57, Suppl. 2, 67-75 (1986).

Protection against mycobacterial disease involves cell-mediated immunity. Joint International Union Against Tuberculosis and World Health Organization Study Group, Tubercle, 63:157-169 (1982); Hahn, H. and S.H.E. Kaufman, Rev. Infect. Dis., 3:1221-1250 (1981). T lymphocytes cloned from patients or from volunteers immunized with killed mycobacteria have been tested for their ability to recognize the recombinant mycobacterial proteins. Lymphocyte-proliferation assays demonstrate that most of the antigens identified with monoclonal antibodies are involved in the T-cell response to mycobacterial infection or vaccination in mice and in humans. Limiting dilution analysis indicates that 20% of the mycobacterial-reactive CD4⁺ T lymphocytes in mice immunized with M. tuberculosis recognize a single protein, the 65-kDa antigen. Kaufman, S.H.E. et al., Eur J. Immunol., 17:351-357 (1987).

### Summary of the Invention

The present invention relates to stress proteins and methods of enhancing an individual's immune response, to a pathogen other than mycobacterium in particular, it relates to the use of such stress proteins for the manufacture of a "vaccine" in immune prophylaxis therapy, which results in an induction or enhancement of immune response to a selected pathogen in immune prophylaxis, stress proteins are administered to prevent or reduce the effects in an individual of a pathogen, which can be any virus, microorganism or other organism or substance (e.g., a toxin or toxoid) which causes disease. In preventing or reducing adverse effects of nonviral pathogens (e.g. , bacteria) according to the method of the present invention, an individual's immune response to the nonviral pathogen's stress protein(s) is induced or enhanced through the administration of a vaccine which includes the pathogen's stress protein(s) and, generally, an adjuvant.
Preventing or reducing adverse effects of viral pathogens, as well as preventing cell transformation or reducing the extent to which it occurs, according to the present method, is effected by transiently enhancing an individual's immune surveillance system. In this instance, the causative pathogens (i.e., virus; transforming agent) do not have stress proteins of their own. Enhancement of immune response can be effected by modulating the immune cells by stimulation with a nonviral stress protein (e.g., a bacterial stress protein) or modulating the individual's stress response by any means (e.g., local application of heat).

In immune therapy, such as is used in treating autoimmune diseases, stress proteins known to be involved in the autoimmune response are administered to turn down an individual's immune response by tolerizing the individual to the stress proteins. See EP-A-0,262,710 Alternatively, the immune response to stress protein, which is known to occur in autoimmune disease, is reduced by interfering with the ability of immune cells which respond to stress proteins to do so.

A selected stress protein of the present invention can be administered to an individual, according to the method of the present invention, and result in an immune response which provides protection against subsequent infection by a nonviral organism (e.g., bacteria, other infectious agents which produce stress proteins).

### Brief Description of the Drawings

Figure 1 is a graphic representation of the homologies between mycobacterial antigens and known stress proteins. Figure 1A is a representation of sequence similarity between portions of the M. tuberculosis 71-kDa antigen (residues 1-204; TB 71kDa) and the E. coli DnaK protein (residues 430-469). Figure 1B is a representation of sequence similarity between portions of the M. tuberculosis 65-kDa antigen (residues 1-540; TB 65kDa) and the E. coli GroEL protein (residues 1-547).

Figure 2 is a comparison of the amino acid sequence of the human P1 protein (573 residues) and the amino acid sequence of the groEL protein (547 residues).

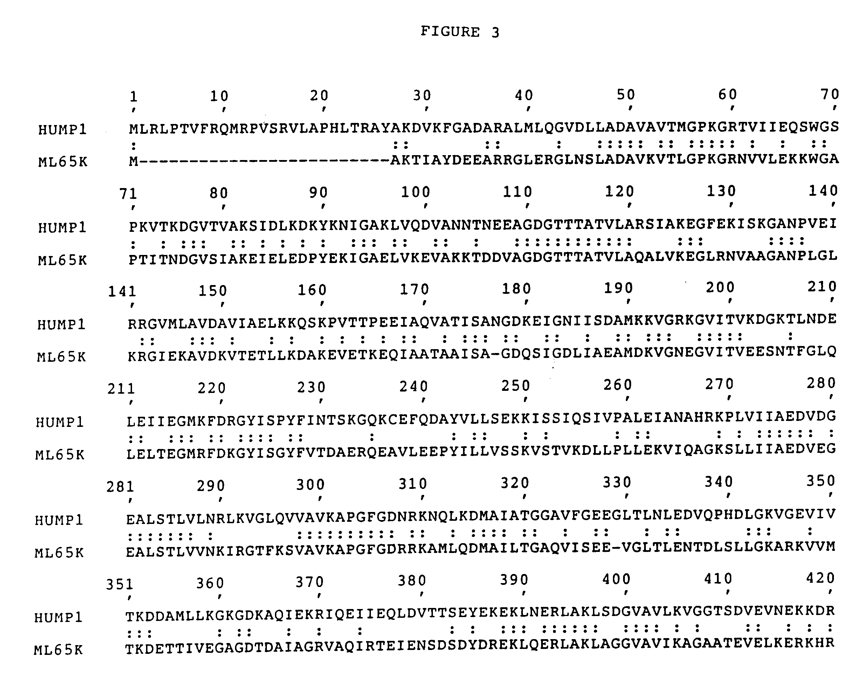

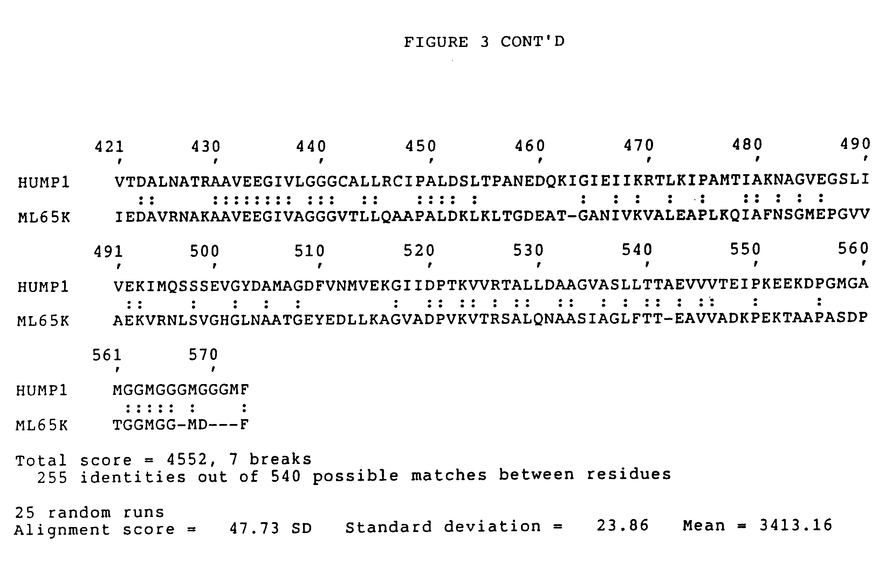

Figure 3 is a comparison of the amino acid sequence of the human P1 protein (573 residues), which is a homolog of groEL protein, and the amino acid sequence of the 65kDa M. leprae protein (540 residues).

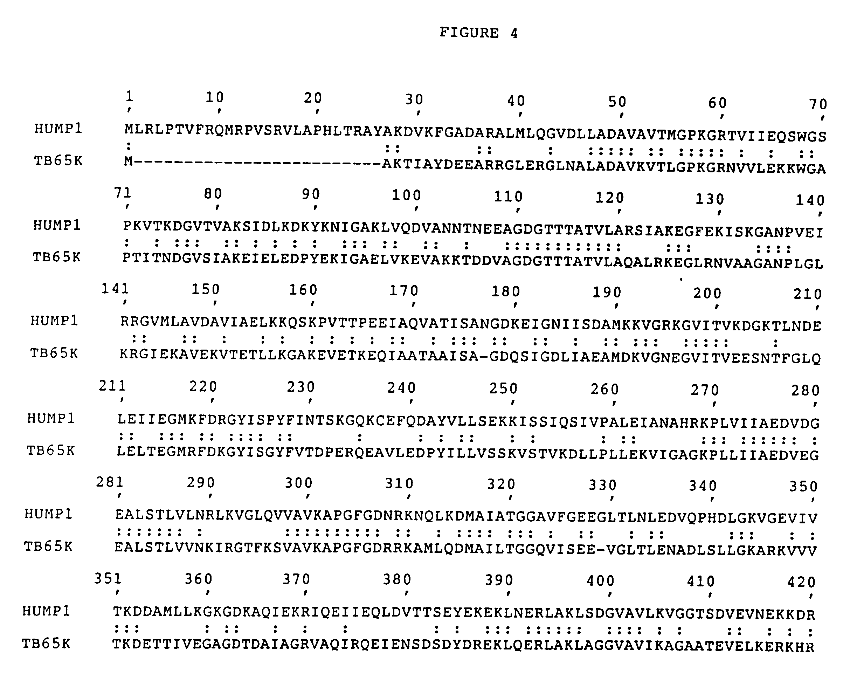

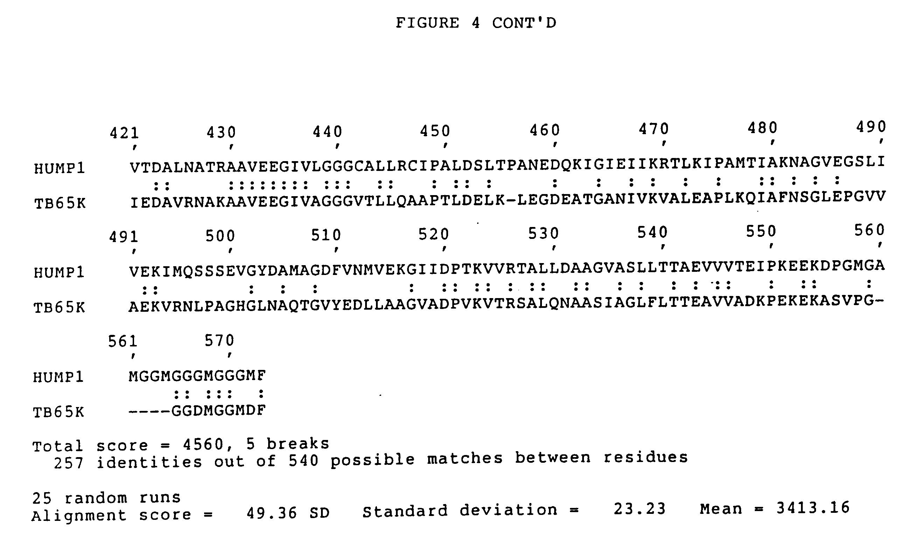

Figure 4 is a comparison of the amino acid sequence of the human P1 protein (573 residues), which is a homolog of the groEL protein, and the amino acid sequence of the 65kDa M. tuberculosis protein (540 residues).

### Detailed Description of the Invention

The present invention is based on the observation that stress proteins are among the major antigens available for presentation to T lymphocytes and may be common immune targets in a broad spectrum of infectious diseases. Immune responses to stress proteins are involved in immune surveillance by the body and a variety of different T cell types has been shown to recognize highly conserved stress protein determinants. Several observations, described below, suggest a model of immune surveillance in which self-reactive T cells provide a first line of defense against infection or other invasion by pathogens and against cell transformation by recognizing and helping to eliminate stressed autologous cells, as well as cells infected with intracellular bacteria. Without wishing to be bound by this model, it is presented as one means by which it is possible to explain why prokaryotic and eukaryotic cells respond to a variety of potentially damaging stimuli, such as elevated temperature, by increasing the synthesis of a family of proteins, referred to as stress proteins, which are among the most highly conserved and abundant proteins found in nature.

Investigation of antigens involved in the immune response to the tuberculosis and leprosy bacilli (M. tuberculosis and M. leprae) initially led to the observation that a variety of stress proteins are among the major targets of the immune response, as is described at greater length below.

Further assessment has demonstrated that stress proteins may be common immune targets in a broad spectrum of infectious diseases. Sequence analysis has revealed 70-kDa heat shock protein homologues among major antigens of the protozoan parasites Plasmodium falciparum (Bianco, A.E. et al., Proc. Natl. Acad. Sci., USA, 83:8713-8717 (1986)) and Schistosoma mansoni (Hedstrom, R. et al., J. Exp. Med., 165:1430-1435 (1987)) and the malarial parasite Brugia malayi (Selkirk, M.E. et al., J. Cell Biochem., 12D:290 (1988)). Similarly, homologues of GroEL have been found among antigens involved in the immune response to Salmonella typhimurium and Coxiella. Vodkin, M.H. and J.C. Williams, J. Bacteriol, 170:1227 (1988). The presence of stress proteins among major immune targets in a variety of human pathogens is support for the idea that the stress response may be a general component of infection and that stress proteins should be considered among candidates for subunit vaccines. All organisms respond to heat by inducing synthesis of heat shock proteins (hsps), which are a group of proteins. This response is the most highly conserved genetic system known and has been shown to occur in every organism, including microorganisms, plants and animals, investigated to date. Many of the characteristics of the response are common to all organisms and the hsps are among the most highly conserved proteins known. For example, hsp90 family and hsp70 family proteins are present in widely diverse organisms. The proteins in each family -- even in such diverse organisms -- show approximately 50% identity at the amino acid level and at the nonidentical residues, exhibit many similarities. Several of the proteins induced by heat are also induced by a variety of other stresses. The hsps or a closely related/similar protein are present in all organisms at normal temperatures and have been shown to have key functions in normal cell metabolism. Lindquist, S. and E.A. Craig, Ann. Rev. Genet., 22:631-677 (1988). Because the stress response is common to prokaryotes and eukaryotes and stress proteins are among the most highly conserved in sequence, it is reasonable to expect that an antigen from one pathogen could immunize against another pathogen. Exposure to foreign stress proteins early in life might, in fact, induce a degree of immunity to a variety of infectious agents. If so, this could provide an explanation for the observation that, for many pathogens, only a fraction of infected individuals actually acquire clinical disease.

The following is a description of the relationship which has been observed between stress proteins and the immune response to mycobacterial infection; of the observation and supporting information that stress proteins are immune targets in many non-viral infections; of recognition of the fact that immune responses to conserved stress protein determinants may play an important role in autoimmune pathology in rheumatoid arthritis, as well as in adjuvant arthritis; and of the role of stress proteins in immune surveillance, as well as a model proposed for immune surveillance in which self-reactive T cells provide a first line of defense against infection and cell transformation.

### Mycobacterial Stress Proteins are Targets of the Immune Response

An intriguing relationship between stress proteins and the immune response to mycobacterial infection has been observed. A more detailed examination of stress protein determinants and immune response mechanisms is essential to understanding the relationships among stress proteins, infection, and immunity.

In view of the involvement of proteins of M. tuberculosis and M. leprae in humoral and cell-mediated immune responses and to establish the functions of the these proteins in the mycobacterial cell, the DNA encoding several of the M. tuberculosis and M. leprae antigens have been sequenced. It has been demonstrated, as a result, that many of these mycobacterial protein antigens exhibit striking sequence similarity to known stress-induced proteins. Three of the M. leprae and two of the M. tuberculosis protein antigens studied have been shown to exhibit striking sequence similarity to known stress proteins. For reasons discussed in the Exemplification, it is concluded that two of the M. leprae and two of the M. tuberculosis antigens are homologues of the E. coli DnaK and GroEL proteins.

In experimental mice, immunization with mycobacterial lysates elicits antibody responses to at least six M. tuberculosis protein antigens and a similar number of M. leprae protein antigens. Monoclonal antibodies specific for these proteins have been used to isolate clones from λgt11 DNA expression libraries of M. tuberculosis and M. leprae. The sequence of the DNA clones revealed that mycobacterial hsp70 (alias 70 kDa antigen) and hsp60 (alias 65 kDa antigen, groEL) were the major targets of the murine antibody response to both M. tuberculosis and M. leprae. Two additional hsp's, an 18 kDa member of the small hsp family and a 12 kDa homologue of groES, were found among the M. leprae and M. tuberculosis antigens. Young, D.B., et al., Proc. Natl. Acad. Sci., USA, 85:4267-4270 (1988); Shinnick, T.M., et al., Nuc. Acids Res., 17:1254 (1989).

The mycobacterial stress proteins are among the immunodominant targets of both murine antibody and T cell responses. In one study which summarized results obtained from 10 laboratories, a collection of 24 murine monoclonal antibodies recognized 6 M. leprae proteins; 7 of these antibodies are directed against 6 different determinants in the M. leprae hsp60. Engers, H.D., et al., Infect. Immun., 48:603-605 (1985); Mehra, V., et al., Proc. Natl. Acad. Sci., USA, 83:7013-7017 (1986). In a similar study, 3 of 33 monoclonal antibodies raised against M. tuberculosis recognized the M. tuberculosis hsp60 protein. Engers, H.D., et al., Infect. Immun., 51:718-720 (1986). Finally, limiting dilution analysis indicates that 20% of the mycobacterial-reactive CD4⁺ T lymphocytes in mice immunized with M. tuberculosis recognize this antigen. Kaufmann, S.H., et al., Eur. J. Immunol., 17:351-357 (1987).

Although a rigorous quantitative analysis of the human immune response to mycobacterial stress proteins has not yet been reported, mycobacterial stress proteins are recognized by human antibodies and T lymphocytes and the evidence suggests that these proteins are among the major targets of the human cell mediated immune response. Emmrich, F., et al., J. Exp. Med., 163:1024-1029 (1985); Mustafa, A.S., et al., Nature (London), 319:63-66 (1986); Oftung, F., et al., J. Immunol., 138:927-931 (1987); Lamb, J.R.,et al., EMBO J., 6:1245-1249 (1987). T lymphocytes from patients with mycobacterial infection or from volunteers immunized with mycobacteria have been cloned and tested for their ability to recognize the mycobacterial stress proteins. In each of these studies, some fraction of the human T cell clones were shown to recognize one or more of the mycobacterial stress proteins.

### Stress Proteins are Immune Targets in Non-viral Infections

The observation that stress proteins are important targets of the immune response to mycobacterial infection and the knowledge that the major stress proteins are conserved and abundant in other organisms suggested that stress proteins are likely to be immune targets in many non-viral infections. Indeed, that is now clearly the case. Antigens from a wide variety of infectious agents have been identified as members of stress protein families. The major stress protein antigen recognized by antibodies in bacterial infections is hsp60. "Common antigen", an immunodominant protein antigen long known to be shared by most bacterial species, turns out to be hsp60. Shinnick, T.M., et al., Infect. Immun., 56:446 (1988); Thole, J.E.R., et al., Microbial Pathogenesis, 4:71-83 (1988). Stress proteins have also been identified as immune targets in most major human parasite infections. Bianco, A. E., et al., Proc. Natl. Acad. Sci., USA, 83;8713 (1986); Nene, V., et al., Mol. Biochem. Parasitol., 21:179 (1986); Ardeshir, F., et al., EMBO J., 6:493 (1987); Hedstrom, R., et al., J. Exp. Med., 165:1430 (1987); Selkirk, M.E., et al., J. Cell Biochem., 12D:290 (1988); Engman, D.M., et al., J. Cell Biochem., 12D: Supplement, 290 (1988); Smith, D.F., et al., J. Cell Biochem., 12D:296 (1988). Antibodies to hsp70 have been identified in the sera of patients suffering from malaria, trypanosomiasis, leishmaniasis, shistosomiasis and filariasis. Hsp90 is also a target of antibodies in trypanosomiasis and a member of the small hsp family is recognized in some patients with shistosomiasis.

### Stress Proteins and Autoimmune Processes

Rheumatoid arthritis is characterized by a chronic proliferative and inflammatory reaction in synovial membranes which is thought to involve autoimmune processes. Rat adjuvant arthritis resembles human rheumatoid arthritis in many respects, and has been used as an experimental animal model for human disease. Pearson, C.M., Arthritis Rheum., 7:80-86 ((1964). Adjuvant arthritis can be induced in rats with a single intradermal injection of killed M. tuberculosis in complete Freund's adjuvant. An autoimmune process involving T lymphocytes appears to be responsible for the generation of the disease. Holoshitz, J., et al., Science, 219:56-58 (1983). T cell lines isolated from the draining lymph nodes of arthritic rats and propagated in vitro by stimulation with M. tuberculosis-pulsed syngeneic antigen presenting cells can cause a transient form of the disease when transferred to irradiated rats. Since care was taken in these experiments to exclude the transfer of contaminating M. tuberculosis, this result strongly suggests that the clinical effects of the disease are a consequence of an autoimmune reaction in which the autoantigen is shared with M. tuberculosis.

The rat and M. tuberculosis antigens recognized by the arthritogenic T cells have been sought for a number of years. A number of different proteins present in synovial membranes have been proposed to be the cross-reactive rat antigen, but were later discounted as procedures for the purification of these proteins improved. van Eden, W., et al., Proc. Natl. Acad. Sci., USA, 82:5117-5120 (1985); Holoshitz, J., et al., Science, 219:56-58 (1983). The M. tuberculosis antigen recognized by the arthritogenic T cells was recently shown to be a 65 kDa protein (van Eden, W., et al., Nature, 331:171 (1988), which has now been shown to be hsp60 (see the Exemplification). Using a combination of truncated recombinant 65 kDa proteins and peptides, a nine amino acid epitope of hsp60 has been identified as the minimum stimulatory sequence for arthritogenic T cell clones in proliferation assays. Now that it is clear that some arthritogenic T cells recognize the mycobacterial hsp60, it is quite possible that the rat autoantigen is also hsp60.

The results obtained in the adjuvant arthritis model led investigators to determine whether T lymphocytes from human rheumatoid arthritis patients also recognize mycobacterial antigens. These investigators have found not only that patients with rheumatoid arthritis have T cells that recognize M. tuberculosis antigens, but that these T cells have diverse phenotypes. Substantial proliferative responses to mycobacterial extracts are observed with uncloned T cells (predominantly CD4⁺) from both synovial infiltrates and peripheral blood, although responses are generally greater in synovial infiltrates. Abrahamson, T.G., et al., Scand. J. Immunol., 7:81-90 (1978); Holoshitz, J., et al., Lancet ii, 305-306 (1986). Holoshitz et al. found that 4 of 5 T cell clones isolated from human rheumatoid synovia which respond to M. tuberculosis antigens were CD4⁻ CD8⁻ cells with γ/δ T cell receptors. Holoshitz, J., et al., Nature, 339:226-229 (1989). This observation is interesting because γ/δ T cells have yet to be assigned a role in immunity. One of the γ/δ clones was tested for its ability to respond to purified mycobacterial hsp60 and was found to be positive in proliferation assays. Due to the conserved nature of stress proteins, these T cells have the potential for autoreactivity. Lamb and coworkers have shown that polyclonal T cells from synovial infiltrates recognize both mycobacterial hsp60 and hsp70. Lamb, J.R., et al., Intl. Immunol., in press (1989). The population of T cells that recognize the mycobacterial stress proteins were shown to respond to E. coli hsp60 and hsp70 and, most interestingly, human hsp70 purified from heat shocked macrophages. Thus, immune responses to conserved stress protein determinants, perhaps initiated by bacterial infection (not necessarily by mycobacteria), may play an important role in autoimmune pathology in rheumatoid arthritis, as well as in adjuvant arthritis.

### Stress Proteins and Immune Surveillance

A variety of different T cell types has now been shown to recognize highly conserved stress protein determinants. The ability of cells to respond to stress by increasing the levels of the highly conserved stress proteins; the presence of T cells of diverse phenotypes in healthy individuals that are capable of recognizing self stress protein determinants; and observations that stress responses are induced by viral infection and by cell transformation, all suggest a model of immune surveillance in which self-reactive T cells provide a first line of defense against infection and transformation by recognizing and helping to eliminate stressed autologous cells, as well as cells infected with intracellular bacteria. The pool of lymphocytes that recognize conserved stress protein determinants might be induced during establishment of natural microbial flora on the skin and in the gut, and maintained by frequent stimulation by bacteria and viruses as well as other stressful stimuli encountered during a normal lifetime. This model is attractive because it provides a way in which the immune system could exploit the existence of conserved epitopes in stress proteins to respond immediately to antigenically diverse pathogens and cellular changes, producing an initial defense that need not await the development of immunity to novel antigens,

Stress protein induction occurs in eukaryotic cells following infection by diverse viruses in vitro. Collins, P.L., and Hightower, L.E., J. Virol., 44:703-707 (1982); Nevins, J.R., Cell, 29:913-939 (1982); Garry, R.F., et al., Virology, 129:391-332 (1988); Khandjian, E.W. and Turler, H., Mol. Cell Biol., 3:1-8 (1983); LaThangue, N.B., et al., EMBO J., 3:267-277 (1984). CTL that recognize these neo-antigens could limit the spread of virus by killing infected cells, possibly before substantial amounts of mature virus are assembled, and by secreting the lymphokine γ-interferon. Pestka, S., in: Methods Enzymol., Interferons, Part A., Vol. 79, Academic Press, New York, pp.667 (1981). Evidence consistent with this idea is emerging. Koga et al., have shown that infection of primary murine macrophages with CMV rendered them susceptible as targets for MHC0I restricted CD8⁺ CTL specific for linear epitopes of M. tuberculosis hsp60. Koga, T., et al. Although the epitope recognized by these CTL on infected macrophages was not defined, it is tempting to speculate that a cross-reactivity with self hsp60 epitopes is being observed. Indeed, the same groups showed that a homologous hsp60 is constitutively present in macrophages and is upregulated by γ-interferon stimulation.

T cells capable of recognizing autologous cells stressed by transformation could help eliminate nascent tumour cells. Stress proteins appear to be produced at high levels in at least some transformed cells. Bensaude, O. and Morange, M., EMBO J., 2:173-177 (1983). An 86 kDA murine tumour antigen has been found to be homologous to representatives of the hsp90 family in yeast and Drosophila. Ullrich, S.J., Proc. Natl. Acad. Sci., USA, 83:3121-3125 (1986). Immunization of mice with the purified protein led to inhibition of tumor growth in 95% of experimental animals that had been seeded with cultured tumor cells. All of the protected mice had high titres of anti-hsp90 serum antibody which was able to precipitate murine hsp90 from lysates of heat shocked mouse embryo cells. Again, a role for autoreactive lymphocytes is implied.

The lymphocytes which recognize conserved stress protein determinants must be capable of discriminating between normal and stressed cells. Since many stress proteins are constitutively expressed in normal cells, although at lower levels than in stressed cells, the potential for autoreactivity is ever-present. Normal cells may escape destruction by expressing only substimulatory levels of stress protein determinants on their surfaces. In addition, stress proteins may only be processed and presented during stress, and it may be relevant that many stress proteins have altered intracellular locations during stress. Finally, immune regulatory networks may prevent activation of autoreactive T cells under normal conditions. The regulatory constraints required by this system might occasionally break down, perhaps during stress caused by bacterial or viral infections, leading to autoimmune disease. Rhematoid arthritis may be such a disease.

### Modulation of Immune Response

The precise relationship between stress proteins and the host immune response to infection is as yet undefined. When cells are subjected to a variety of stresses, they respond by selectively increasing the synthesis of a limited set of stress proteins. Some stress proteins, including the products of dnaK and groEL, are major constituents of the cell under normal growth conditions and are induced to even higher levels during stress. Lindquist, S., Annu. Rev. Biochem., 55: 1151-1191 (1986); Neidhardt, F.C. and R.A. VanBogelen, In Escherichia coli and Salmonella Typhimurium, Cellular and Molecular Biology, (eds. Neidhardt, F.C., Ingraham, J.L. Low, K.B. Magasanik, B. Schaechter, M. and Umbarger, H.E.) Am. Soc. Microbiol., Washington, D.C., pp. 1134-1345 (1987). It has now been demonstrated that stress-response proteins are targets of the immune response. It is reasonable to expect that immunodominant antigens would be found among such abundant proteins, as has been shown to be the case.

Stress-induced proteins or their functional equivalents, can be used to immunize an animal against a nonviral infection or, alternatively, other selected stress-induced proteins, or their functional equivalents, can be used to induce immune tolerance in an animal. According to the method of the present invention, it is possible to modulate the immune response in an individual by altering the individual's response to stress proteins. In particular, it is possible to modulate an individual's response to a pathogen (e.g. , bacterium, virus, or other organism or agent, such as parasites, toxins, toxoids, which causes disease or cell transformation).

It is possible to use stress proteins, as described herein, as a vaccine which, after administration to an individual, produces or enhances an immune response in that individual, providing protection against subsequent infection. Because, as demonstrated, these are proteins in which there are regions of highly conserved amino acid sequences and such proteins have been shown to be ubiquitous targets in mycobacterial, bacterial and other infections, they can be used to elicit an equally ubiquitous immune response.

For example, in the case of a pathogen which expresses stress proteins, such as nonviral pathogens, two approaches to enhancing an individual's immune response (and, thus, reducing the pathogen's effects) can be used.

First, because the nonviral pathogen's stress proteins are distinguishable from those of the host, it is possible to induce an immunoprophylactic response specific to the pathogen's stress proteins. This can be carried out by administering a vaccine which includes all or a portion (e.g., sufficient sequence to have the desired stimulatory effect on immune response) of the pathogen's stress protein or of another protein having an amino acid sequence sufficiently similar to that of the stress protein sequence to stimulate the immune response to the pathogen. Alternatively, highly conserved stress protein determinants, such as those shown to be recognized by a variety of T cells, can be administered as a type of "general" vaccine. In either case, the immune response to the stress protein sequence will be increased and effects of the nonviral pathogen will be reduced (decreased, prevented or eliminated).

Second, it is also possible to induce or enhance the immune surveillance system or response which is directed to stressed host cells. This is described further in the context of enhancing immune response in those instances in which the pathogen (e.g., a virus, transforming agent) does not have (express) its own stress proteins (i.e., stress proteins distinguishable from host stress proteins).

The vaccine administered to induce or enhance immune response to nonviral pathogens includes a stress protein of the pathogen against which an immune response is desired, a portion of that protein of sufficient size to stimulate the desired immune response or a protein or amino acid sequence (e.g., a polypeptide) which is the functional equivalent of the pathogen stress protein in that it is sufficiently homologous in sequence to that of the stress protein to be capable of eliciting the desired response. The term "sufficiently homologous in sequence to that of the stress protein" means that the sequence of the protein or polypeptide will generally show approximately 50% identity with the stress protein amino acid sequence. The vaccine can also include an adjuvant, an appropriate carrier and an appropriate buffer. The protein or amino acid sequence present in the vaccine can be produced using known techniques. For example, it can be obtained (isolated) from a source in which it occurs in nature, can be produced by cloning and expressing the gene encoding the desired stress protein or stress protein portion or can be synthesized chemically or mechanically.

In the case of a pathogen, such as a virus or a transforming agent (i.e., an agent whose activity results in production or formation of cancer cells), which does not express its own stress proteins, the following approach is used to enhance immune response or normal immune surveillance (i.e., the ability of the immune system to recognize self as well as foreign proteins). A vaccine which includes a bacterial, mycobacterial or other stress protein can be administered. Although there are no viral stress proteins to be used for this purpose, administration of such a vaccine will enhance the existing immune surveillance system. In addition, immune surveillance can also be enhanced by applying local heat or any other substances or changes in condition which induce the stress response in the individual being treated. (This can also be employed in conjunction with the vaccine, described previously, administered to enhance immune response to a stress protein-producing pathogen.) It is known that increased levels of stress proteins are produced in many types of cancer cells. Enhancement of the immune surveillance system, as described, can be used to facilitate destruction and/or to prevent progression or establishment of cancer cells.

The present invention is further illustrated by the following exemplification, which is not intended to be limiting in any way.

### EXEMPLIFICATION

Recombinant DNA Clones. The isolation and characterization of M. tuberculosis and M. leprae λgt11 genomic DNA clones with murine monoclonal antibodies have been described. Husson, R.N. and Young, R.A., Proc. Natl. Acad. Sci., USA, 84:1679-1683 (1987); Young, R.A., et al., Nature, (London) 316:450-452 (1985). DNA was isolated from these clones and was manipulated by standard procedures. Davis, R.W., Advanced Bacterial Genetics: A Manual for Genetic Engineering (Cold Spring Harbor Lab., Cold Spring Harbor, NY), (1980).
DNA Sequence Analysis. DNA was subcloned into vector M13mp18 or M13mp19 (New England Biolabs), as suggested by the supplier. Dideoxynucleotide chain-termination reactions and gel electrophoresis of the sequenced produced were as described. Davis, R.W., Advanced
Bacterial Genetics: A Manual for Genetic Engineering (Cold Spring Harbor Lab., Cold Spring Harbor, NY), (1980). DNA sequences were determined for both strands of DNA. Computer analysis of sequences with UWGCG programs was as described by Devereux, J., et al. Nucleic Acids Res., 12:387-395 (1984).
Immunoblot Analysis. Escherichia coil strain TG1 was transformed with the following plasmids by standard procedures (Maniatis, T., et al., Molecular Cloning, A Laboratory Manual (Cold Spring Harbor Lab., Cold Spring Harbor, NY) (1982), with selection for ampicllin resistance: pND5, a derivatrive of pBR325 containing the E. coli groE genes (Jenkins, A.J., et al., Mol. Gen. Genet, 202:446-454 (1986); pUC8 (Vic?, J., Gene, 19:259-268 (1982); pUC8 with insert DNA for λgt11 clone Y3178 (M. leprae 65-kDa antigen, Young, R.A., et al., Nature, (London) 316:450-452 (1985)) ligated in the EcoRI site.

Overnight cultures of E. coli strains in Luria-Bertani (LB) medium were centrifuged and resuspended in isotonic phosphate-buffered saline at a cell density corresponding to an absorbance of 20 at 60 nm. An equal volume of sample buffer containing 2% (wt/vol) polycrylamide gels in the presence of NaDodSO₄ was added, and, after heating on a boiling water bath for 2 min, 5-ml samples were electrophoresed on 12% (wt/vol) polycrylamide gels in the presence of NaDodSO₄. Blots were prepared by electrophoretic transfer of the proteins to a nitrocellulose membrane, and binding of monoclonal antibodies was assayed with a peroxidase-conjugated secondary antibody as described. Young., D.B.. et al., Infect. Immun., 55:1421-1425 (1987).

Six M. tuberculosis and six leprae proteins have been implicated in the immune response to the mycobacterial pathogens (T1). To obtain clues to the normal cellular function of several of these mycobacterial antigens, DNA clones encoding these proteins, isolated by using monoclonal antibodies to probe lambdagt11 libraries (Husson, R.N. and Young, R.A., Proc. Natl. Acad. Sci., USA, 84:1679-1683 (1987); Young, R.A., et al., Nature, (London) 316:450-452 (1985)) were subjected to sequence analysis. The sequences elucidated have been submitted to the GenBank sequence database.

The Mycobacterial 71-k Da Antigen. The 71-k Da antigen of M. tuberculosis is recognized by human T cells during infection (Table).

**TABLE**

| Mycobacterial protein antigens | | | |
|---|---|---|---|
| Protein, kDA | Recognized by human T cells | Subjected to sequence analysis | Homology with known proteins |
| M. tuberculosis | | | |
| 71 | + | + | DnaK |
| 65* | + | + | GroEL |
| 38 | + | - | - |
| 19 | + | + | None |
| 14 | + | - | - |
| 12 | ND | - | - |

| M. leprae | | | |
|---|---|---|---|
| 70 | ND | - | DnaK |
| 65 | + | + | GroEL |
| 36 | + | - | - |
| 28 | + | - | - |
| 18 | + | + | Plant Hsp |
| 12 | ND | - | - |
| Mycobacterial protein antigens, their recognition by human T cells, and homology of the deduced mycobacterial protein sequences to known proteins are summarized. ND, not determined; +, yes; -, no | | | |

| | | | |
|---|---|---|---|
| * Includes data derived from study of the 65-kDA antigens of M. bovis BCG (bacillus Calmette-Guerin), which is identical to the M. tuberculosis 65-kDA antigen. | | | |
| + A. S. Mustafa, J. R. Lamb, D. Young and R. A. Young, unpublished data. | | | |

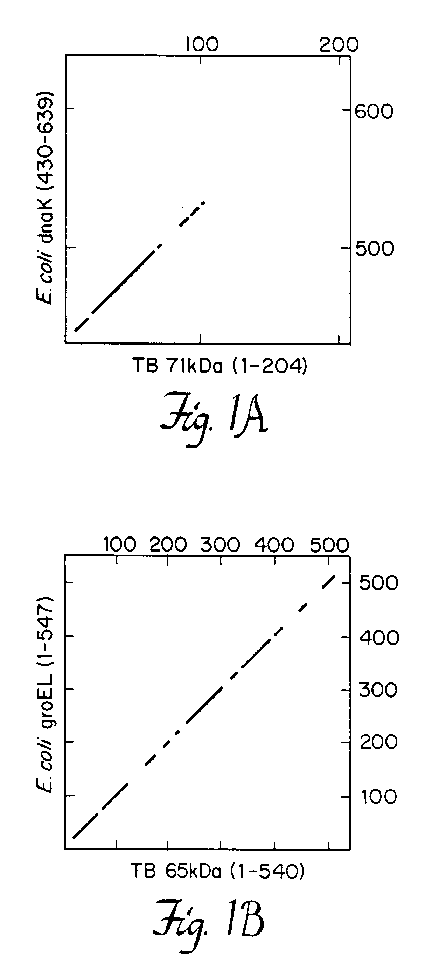

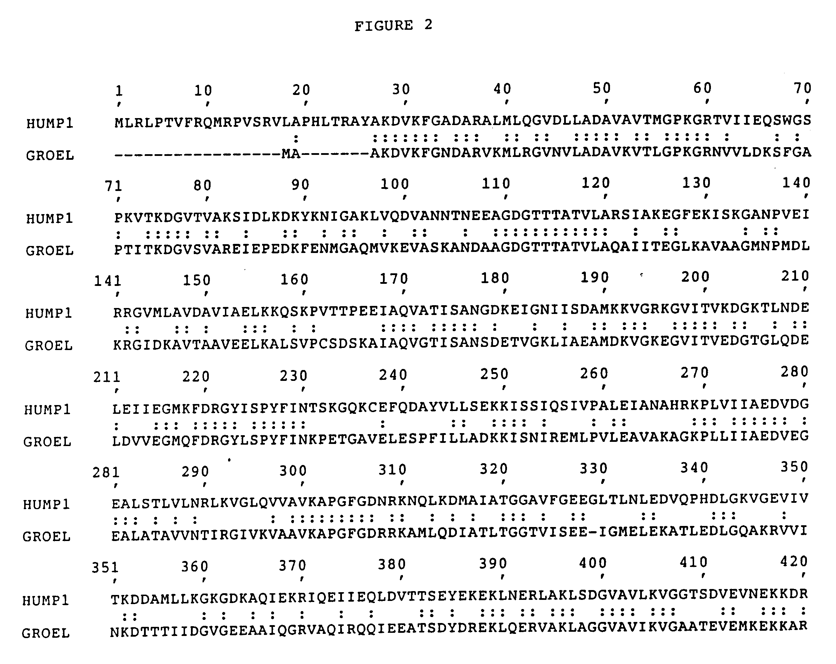

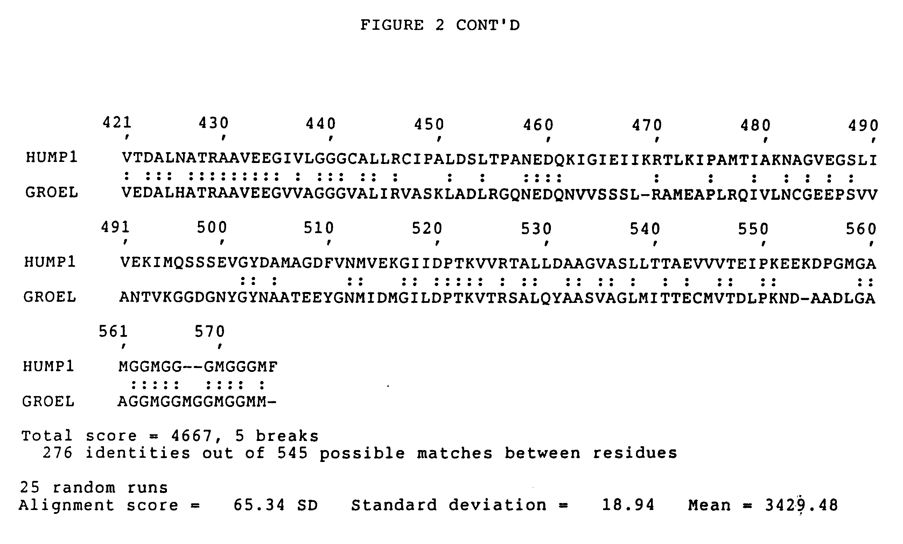

The insert DNA of lambdagt11 clone Y3271 (Husson, R.N. et al., Proc. Natl. Acad. Sci., USA, 84:1679-1683 (1987). was sequenced to obtain amino acid sequence information for the 71-kDa antigen of M. tuberculosis. This clone produces a beta-galactosidase fusion protein containing the carboxyl-terminal one-third of the 71-kDa antigen exhibits 40% amino acid sequence identity with the comparable segment of the dnaK gene product from E coli (Bardwell, J.C., et al., Proc. Natl. Sci., USA, 81:848-852 (1984)), (Fig. 1). Fig 2A shows the extent of sequence similarity between portions of the mycobacterial and the E. coli 70-k Da polypeptides. Sequences transcriptionally downstream from the mycobacterial 71-k Da gene predict a 356-amino acid protein homologous to the E. coli dnaJ gene product (unpublished data), indicating that the E. coli dnaK-dnaJ operon structure is conserved in M. tuberculosis and consistent with the conclusion that the mycobacterial 71-kDa antigen is a homologue of the E. coli dnaK gene product. The product of the dnaK gene is a member of the 70-kDa heat shock protein family that is highly conserved among prokaryotes and eukaryotes (Bardwell, J.C., et al., Proc. Natl. Sci., USA, 81:848-852 (1984); Lindquist, S., Annu. Rev. Biochem., 55:1151-1191 (1986).

The M. leprae 70-k Da antigen cross-reacts with monoclonal antibodies directed to the M. tuberculosis and M. leprae are both members of the 70-k Da heat shock protein family of stress proteins.

The Mycobacterial 65-kDa antigen. The 65-kDa antigens of M. tuberculosis and M. leprae are involved in in the human T-cell response to mycobacterial infection (Table). Genes encoding these proteins have been isolated (Husson, R.N. and Young, R.A., Proc. Natl. Acad. Sci., USA, 84:1679-1683 (1987); Young, R.A., et al., Nature, (London) 316:450-452 (1985)) and sequenced (Shinnick, T.M., J. Bacteriol., 169:1080-1088 (1987); Mehram, V., et al., Proc. Natl. Acad. Sci., USA, 83:7013-7017 (1986)), revealing that the amino acid sequences of the 65-kDa antigens of M. tuberculosis and M. leprae are 95% identical. These protein sequences exhibit no significant sequence similarity to proteins in the GenBank database.

Identification of these proteins was based on the observation that some monoclonal antibodies directed against the mycobacterial 65-kDa antigens cross-react with an E. coli protein of 60kDa. E. coli cells transformed with the plasmid pND5 (Sanger, F., et al., Proc. Natl. Acad. Sci., USA, 74:5463-5467 (1977), which contains the E. coli gro E genes, had been shown to accumulate large amounts of the 60-kDa protein. A comparison of the mycobacterial 65-kDa protein sequences with those determined for E. coli groEL (C. Woolford, K. Tilly, C. Georgopoulous, and R.H., unpublished data) revealed the extent of the sequence similarity as shown in Fig. 1B.

The 60-kDa Gro EL protein is a major stress protein in E. coli. Lindquist, S., Annual Rev. Biochem., 55:1151-1191 (1986); Nature, 333:330-334 (1988). There is some evidence that the mycobacterial 65-kDa proteins accumulate in response to stress: Mycobacterium bovis BCG (bacillus Calmette-Guerin) cultures grown in zinc-deficient medium are substantially enriched in this protein (De Bruyn, J., et al., Infect. Immun., 55:245-252 (1987)). This infers that the 65-kDa proteins of M. tuberculosis and M. leprae are homologues of the E. coli Gro EL protein.

Other Mycobacterial Antigens. T lymphocytes that respond to the M. tuberculosis 19-kDa antigen and the M. leprae 18-kDa antigen have been observed in humans with tuberculosis and leprosy, respectively (Table 1). DNA encoding these antigens was sequenced from the Agt11 clones Y3148 (Husson, R.N. and Young, R.A., Proc. Natl. Acad. Sci., USA, 84:1679-1683 (1987); and Y3179 (Young, R.A., et al., Nature, (London) 316:450-452 (1985)), respectively. The M. tuberculosis 19-kDa protein sequence predicted from the DNA exhibited no significant sequence similarity to proteins in the GenBank database.

However, the M. leprae 18-kDa protein sequence was similar to the soybean 17-kDa heat shock protein, a protein representation of a major class of plant heat shock proteins (Schoffl, F. and Van Bogelen, R.A., In: Escherichia coli and Salmonella typhimurium, Cellular and Molecular Biology, Am. Soc. Microbiol., Washington, D.C. (1987).

## Claims

1. Use of all or a portion of a stress protein (or all or a portion of a protein having an amino-acid sequence sufficiently homologous to the amino-acid sequence of the stress protein so that its immune, therapeutic and/or prophylactic activity is retained) for the preparation of a vaccine for enhancing in an individual the immune response to a pathogen (e.g. a non-viral pathogen) other than a mycobacterium.

2. Use of claim 1 in which the vaccine is for immunizing a patient (e.g. an animal) against infection by a pathogen other than a mycobacterium.

3. Use of all or a portion of a stress protein (or all or a portion of a protein having an amino-acid sequence sufficiently homologous to the amino-acid sequence of the stress protein so that its immune, therapeutic and/or prophylactic activity is retained) for the manufacture of a composition for producing or enhancing in an individual an immune response to a disease, other than a mycobacterial disease.

4. Use of claim 3 wherein the disease is selected from malaria, trypanosomiasis, leishmaniasis, shistomaniasis, filariasis and cancer.

5. Use of any one of the preceding claims wherein the stress protein is a member of the hsp90, hsp70, hsp60 or small molecular weight hsp stress protein family.

6. Use of any one of claims 1 to 4 in which the stress protein is selected from E. coli DnaK, GroEL, GroES and DnaJ proteins.

7. Use of any one of claims 1 to 4 in which the stress protein is a mycobacterial stress protein.

8. Use of claim 7 in which the stress protein is selected from M. tuberculosis 71, 65 and 12kDa proteins and M. leprae 70, 65 and 18kDa proteins.

## Patentansprüche

1. Verwendung eines ganzen oder eines Teils eines Streßproteins (oder eines ganzen oder eines Teils eines Proteine mit einer Aminosäuresequenz, die hinreichend homolog zu der Aminosäuresequenz des Streßproteins ist, so daß seine immune, therapeutische und/oder prophylaktische Aktivität beibehalten wird) zur Herstellung eines Impfstoffes zur Verstärkung der Immunantwort in einem Individuum gegen ein Pathogen (z. B. ein nicht-virales Pathogen) außer einem Mycobakterium.

2. Verwendung gemäß Anspruch 1, in welcher der Impfstoff zur Immunisierung eines Patienten (z. B. eines Tiers) gegen eine Infektion eines Pathogens außer einem Mycobakterium dient.

3. Verwendung eines ganzen oder eines Teils eines Streßproteins (oder eines ganzen oder eines Teils eines Proteine mit einer Aminosäuresequenz, die hinreichend homolog zu der Aminosäuresequenz des Streßproteins ist, so daß seine immune, therapeutische und/oder prophylaktische Aktivität beibehalten wird) zur Herstellung einer Zusammensetzung zur Bildung oder Verstärkung einer Immunantwort in einem Individuum gegen eine Erkrankung außer einer mycobakteriellen Erkrankung.

4. Verwendung gemäß Anspruch 3, worin die Erkrankung Malaria, Trypanosomiasis, Leishmaniose, Schistosomiasis, Filariose oder Krebs ist.

5. Verwendung gemäß einem der vorausgehenden Ansprüche, worin das Streßprotein ein Vertreter der hsp90, hsp70, hsp60 oder der hsp Streßprotein-Familie mit geringem Molekulargewicht ist.

6. Verwendung gemäß einem der Ansprüche 1 bis 4, in welcher das Streßprotein aus den E. coli Dnak, GroEl, GroES und DnaJ Proteinen gewählt wird.

7. Verwendung gemäß einem der Ansprüche 1 bis 4, in welcher das Streßprotein ein mycobakterielles Streßprotein ist.

8. Verwendung gemäß Anspruch 7, in welcher das Streßprotein aus M. tuberculosis 71, 65 und 12kDa Proteinen und M. leprae 70, 65 und 18kDa Proteinen gewählt wird.

## Revendications

1. Utilisation d'une protéine de stress entière ou d'une portion de celle-ci (ou d'une protéine entière ou d'une portion de celle-ci dont la séquence en acides aminés est suffisamment homologue à la séquence en acides aminés de la protéine de stress pour que son activité immunitaire, thérapeutique et/ou prophylactique soit conservée) pour la préparation d'un vaccin augmentant chez un individu la réponse immunitaire à un agent pathogène (par exemple un pathogène non viral) autre qu'une mycobactérie.

2. Utilisation selon la revendication 1, dans laquelle le vaccin est destiné à immuniser un patient (par exemple un animal) contre une infection par un agent pathogène autre qu'une mycobactérie.

3. Utilisation d'une protéine de stress entière ou d'une portion de celle-ci (ou d'une protéine entière ou d'une portion de celle-ci dont la séquence en acides aminés est suffisamment homologue à la séquence en acides aminés de la protéine de stress pour que son activité immunitaire, thérapeutique et/ou prophylactique soit conservée) pour la fabrication d'une composition déclenchant ou augmentant chez un individu une réponse immunitaire à une affection, autre qu'une affection d'origine mycobactérienne.

4. Utilisation selon la revendication 3, dans laquelle l'affection est la malaria, la trypanosomiase, la leishmaniose, la schistosomiase, la filariose ou le cancer.

5. Utilisation selon l'une quelconque des revendications précédentes, dans laquelle la protéine de stress est un membre de la famille des protéines hsp 90, hsp 70, hsp 60 ou des protéines de stress hsp de bas poids moléculaire.

6. Utilisation selon l'une quelconque des revendications précédentes, dans laquelle la protéine de stress est choisie parmi les protéines de E. Coli Dnak, GroEL, GroES et DnaJ.

7. Utilisation selon l'une quelconque des revendication 1 à 4, dans laquelle la protéine de stress est une protéine de stress mycobactérienne.

8. Utilisation selon la revendication 7, dans laquelle la protéine de stress est choisie parmi les protéines de 71, 65 et 12 kDa de M. Tuberculosis et les protéines de 70, 65 et 18 kDa de M. Leprae.
